# EUROPEAN PATENT APPLICATION

(11) **EP 1 186 671 A2**
(43) Date of publication of application: **13.03.2002**
(21) Application number: 01121185.1
(22) Date of filing: 04.09.2001
(51) Int. Cl.: C12Q 1/68

(54) **Method for hybridization of arrays on siliceous surfaces**

(30) Priority: 05.09.2000 US 655482
(71) Applicant: Agilent Technologies Inc. (a Delaware Corporation), Palo Alto, CA 94306-2024 (US)
(72) Inventor: Shannon, Karen W., Los Gatos, CA 95030 (US); Lefkowitz, Steven M., Millbrae, CA 94030 (US)
(74) Representative: Schoppe, Fritz, Dipl.-Ing.

(57) **Abstract**

A method (100, 200) of hybridizing arrays of nucleic acids an surface-derivatized siliceous substrates with other nucleic acid materials provides an envelope of conditions to produce sensitive, selective detection of nucleic acid targets, while preserving the intactness of the derivatized surface of the array. The envelope of hybridization conditions includes a hybridization solution having a pH between pH 5.5 and 6.7 and a high hybridization or incubation temperature between 55°C and 70°C. In one embodiment (100), the hybridization solution is maintained (102) at the pH between pH 5.5 and 6.7 and the array is incubated (104) with a nucleic acid material in the pH-maintained hybridization solution at the hybridization temperature of between 55°C and 70°C. The pH of the hybridization solution is maintained (102) with a buffer having a useful buffering capacity between pH 5.5 and 6.7. In another embodiment (200), a nucleic acid material is combined (202) with the hybridization solution at the pH between pH 5.5 to 6.7 containing a buffer and a monovalent cation and the combined solution is incubated (204) with the array at the hybridization temperature of between 55°C and 70°C so as to hybridize the nucleic acid material. Typical hybridization times can range from less than 2 hours to more than 48 hours. The present method is particularly useful for hybridization assays an silylated-siliceous substrates where the incubation time is much greater than about 6 hours at the high hybridization temperature. The envelope of hybridization conditions provide optimized assay performance while maintaining the integrity of the derivatized surface of the siliceous substrate. A kit comprises a microarray having a siliceous substrate with a derivatized surface and an oligonucleotide populated an the surface for hybridizing a another oligonucleotide material. The kit further includes instructions for using the microarray in accordance with the method (100, 200) of the present invention.

## Description

### TECHNICAL FIELD

This invention relates to biological assays. In particular, the invention relates to the materials and methods for assays of arrays of nucleic acid molecules for analytical, therapeutic and diagnostic purposes.

### BACKGROUND ART

Microarrays of DNA or RNA polynucleotides or oligonucleotides are state-of-the-art biological tools used in the investigation and evaluation of genes for analytical, diagnostic, and therapeutic purposes. Microarrays typically comprise a plurality of oligomers, synthesized or deposited on a glass support or substrate in an array pattern. The support-bound oligomers are called "probes", which function to bind or hybridize with a sample of DNA or RNA material under test, called a "target" in hybridization experiments. Note that some investigators also use the reverse definition, referring to the surface-bound oligonucleotides as targets and the solution sample of nucleic acids as probes. Further, some investigators bind the target sample under test to the microarray substrate and put the oligomer probes in solution for hybridization. Either of the "target" or "probes" may be the one which is to be evaluated by the other (thus, either one could be an unknown mixture of polynucleotides to be evaluated by binding with the other). All of these iterations are within the scope of this discussion herein. In use, the array surface is contacted with one or more targets under conditions that promote specific, high-affinity binding of the target to one or more of the probes. The targets are typically labeled with an optically detectable label, such as a fluorescent tag, so that the hybridized targets and probes are detectable with scanning equipment. DNA array technology offers the potential of using a multitude (hundreds of thousands) of different oligonucleotides to analyze changing mRNA populations.

There are numerous types of substrates used in hybridization assays. Common substrates or supports used for array assays are surface-derivatized siliceous substrates, such as glass. DNA microarrays are typically, but not always, synthesized or deposited onto these substrates. The substrate surface is derivatized to enable or facilitate the initial attachment of nucleic acids to the surface for the manufacture of the array probes. Surface derivatization techniques are well-known in the art. A common surface derivatization is silane-based, as further described below (see for example, the cited references in the Definitions section, infra).

Arrays of oligomer probes, such as oligonucleotides or polynucleotides, are hybridized using conventional methods and hybridization solutions. J. Sambrook, E. F. Fritsch, T. Maniatis, Molecular Cloning: A Laboratory Manual, Cold Spring Harbor Laboratory Press, Cold Spring Harbor, Ed. 2^{nd}, 1989, vol. 1-3, incorporated herein by reference, describe the considerations and conditions for hybridization of oligonucleotide probes. Probe length, hybridization temperature, as well as other factors, that are well known in the art affect hybridization conditions. Typically, hybridizations using synthetic oligomers are usually carried out under conditions that are 5-10°C below the calculated melting temperature T_{*m*} of a perfect hybrid to minimize mismatched or non-Watson/Crick base pairing between the probe and target, and maximize the rate at which Watson/Crick base pairs form. Other factors influencing the rate of hybrid formation include the salt concentration, the presence of solvents or co-solvents, the concentration of nucleic acid in solution, the length of hybridization, and the degree and method of agitation.

The hybridization solution typically comprises a salt (monovalent cation), a buffer that provides buffering capacity between pH 6.8-8.5 (more typically between pH 7.0-7.5), a divalent cation chelating agent (e.g. ethylenediaminetetraacetic acid, EDTA), and agents for blocking non-specific binding of targets to the array surface (surfactants, proteins and/or carrier DNA from an organism unrelated to the experiment at hand). A typical hybridization solution contains 6x SSPE (0.9 M NaCl, 60 mM sodium phosphate (pH 7.4); 6 mM EDTA); or 6x SSC (0.9 M NaCl, 90 mM sodium citrate (pH 7.0)), 0.5% w/v sodium dodecyl sulfate (SDS); 100 µg/ml denatured, fragmented salmon sperm DNA; and 0.1% nonfat dried milk.

The array is hybridized for a period of time ranging from about 2 hours to about 2 days, depending on at least the make-up of the probes (i.e., probe length and diversity of probe composition) and the complexity of the target, for example, at a controlled temperature, which typically ranges from 20°C to 70°C, depending on the melting temperature T_{*m*}, as discussed above. Low temperature hybridizations are performed at about 20°C to about 50°C (typically about 37-45°C). High temperature hybridizations are performed at or around 55°C to about 70°C (typically 60°C to 65°C). However, for most nucleic acid microarrays, high temperature hybridizations are preferred in the art since the higher temperature maximizes the rate of Watson/Crick base pairing of nucleotides. The typical time period for hybridization of an array is overnight or longer (i.e., anywhere from 8 hours to at least 24 hours) so as to hybridize the target. The array is then washed and dried and optically scanned to measure the degree of hybridization using conventional methods and equipment that are well known in the art.

A problem in the DNA microarray hybridization art is sporadic poor hybridization assay performance characterized by low-intensity or missing features on the microarray substrate, high backgrounds, and visually "blotchy" substrates. This problem has been observed using conventional hybridization conditions, such as with a solution comprising 10 mM Tris buffer at pH 7.6, 1 M NaCl, and 0.5% N-lauryl sarcosine surfactant at high hybridization temperature of about 66°C and a hybridization time of about 14 to 18 hours. However, the poor performance characteristics have been observed within as little as 6 hours of incubation time at high temperature and conventional buffer solution.

Thus, it would be advantageous to have materials, conditions and methods of hybridizing DNA microarrays on siliceous substrates in biological assays at the preferred higher hybridization temperature range and longer hybridization times without affecting the hybridization assay performance.

### SUMMARY OF THE INVENTION

The present invention provides a method for hybridizing nucleic acid microarrays with other nucleic acid materials used in high throughput analytical, therapeutic, and diagnostic applications. The method uses an envelope of hybridization conditions, which are advantageously compatible with siliceous substrates and surface-derivatized siliceous substrates, for performing assays at high hybridization temperatures for long periods of time. The present invention works particularly well on silane-derivatized siliceous substrates ("silylated-siliceous" substrates). The envelope of conditions addresses solution pH and buffer type, salt composition, surfactant composition, temperature and time. The present invention allows sensitive, selective detection of nucleic acid targets, while preserving the intactness of the derivatized siliceous surface. The method of the invention overcomes the problems found in the art by minimizing substrate surface dissolution when performing assays on derivatized siliceous substrates at high hybridization temperatures between about 55°C and 70°C for time periods used to hybridize a target material. The method of the present invention provides optimized hybridization assay performance by maintaining the integrity of the derivatized surface of the siliceous substrates.

In one aspect of the invention, a method of hybridizing nucleic acid microarrays on surface derivatized-siliceous substrates with another nucleic acid material is provided. The method comprises the steps of maintaining a hybridization solution at a pH between pH 5.5 and 6.7, and incubating the nucleic acid microarray with the nucleic acid material in the pH-maintained hybridization solution at a hybridization temperature ranging from about 55°C to about 70°C. In one embodiment of the method of hybridizing, the hybridization solution is maintained at a pH between pH 5.5 and 6.7 with a buffer having buffering capacity between pH 5.5 and 6.7 and a monovalent cation. Preferably, the pH is maintained between pH 6.0 and 6.6; the buffer is selected from one or more of MES or MOPS; and the monovalent cation is provided by a salt preferably selected from one or more of LiCl, NaCl, or KCl. The hybridization temperature preferably ranges from about 60°C to 66°C. In another embodiment, the hybridization solution further comprises one or more of a chelating agent and a surfactant.

A hybridization assay may take anywhere from less than about 2 hours to more than 48 hours, depending on at least the type and complexity of the hybridization experiment. The problem in the art with dissolution of surface derivatized-siliceous substrates is more evident for hybridizations taking about 8 hours and more to complete at high hybridization temperatures. By "complete", it is meant that a desired amount of hybridization of the target nucleic acid material is achieved, which is both user and target material dependent. The present method may be used for hybridizations times ranging from less than about 2 hours to at least 48 hours. More importantly, the present invention works well where the conventional hybridization parameters fail, i.e., at a hybridization time of about 6 hours and longer. The present invention works particularly well for hybridizations taking at least 24 hours to complete, and more particularly, for hybridizations taking between about 12 hours to at least 24 hours.

In another aspect of the present invention, a method of hybridizing a microarray of an oligonucleotide bound to a silylated-siliceous substrate with another oligonucleotide material is provided. The method of hybridizing comprises the steps of combining the oligonucleotide material with a hybridization solution having a pH between pH 5.5 and 6.7 comprising a buffer and a monovalent cation; and incubating the material in the buffered solution with the oligonucleotide microarray at a hybridization temperature ranging from about 55°C to about 70°C so as to hybridize the oligonucleotide material. As mentioned above, the incubation time period can range from less than about 2 hours to at least 48 hours. The method is particularly useful where conventional hybridization parameters fail on silylated-siliceous substrates at high temperature. Preferably, the buffer is selected from one or more of MES or MOPS, which has a buffering capacity within the pH range of pH 5.5 and 6.7. The monovalent cation is provided by a salt, preferably selected from one or more of LiCI, NaCl, or KCl. Moreover, the hybridization temperature preferably ranges from about 60°C to 66°C. More preferably, the hybridization solution further comprises one or more of a chelating agent and a surfactant.

In still another aspect of the invention, a kit is provided that comprises a microarray of an oligonucleotide on a derivatized surface of a siliceous substrate and instructions for performing a hybridization assay using the microarray. The instructions comprise the method of the present invention. In one embodiment, the kit further comprises a hybridization solution having a pH maintained between pH 5.5 and 6.7, preferably using a buffer having a useful buffering capacity in that pH range.

The method of the present invention overcomes the problems in the art by minimizing degradation of the surface derivatization of the siliceous substrate and further, etching of the substrate, both of which impacted the hybridization results between nucleic acid probes and target materials at high temperatures. Further, the present invention does not affect the signal intensities of the signal labeling system used in conventional hybridization assays. The hybridization conditions and methods of the present invention are particularly useful in DNA or RNA microarray assays performed at high temperatures, i.e., above about 55°C, and for longer hybridization times, i.e., greater than about 6 hours, where conventional buffer compositions, including but not limited to SSC, SSPE, Tris Cl, having high pH (i.e., pH ≥ 6.8) buffering capacity react with the conventional siliceous substrates that are derivatized, and affect hybridization results.

### BRIEF DESCRIPTION OF THE DRAWINGS

The various features and advantages of the present invention may be more readily understood with reference to the following detailed description and Examples taken in conjunction with the accompanying drawings, where like reference numerals designate like structural elements, and in which:
Figure 1 illustrates a flow chart of the method of the present invention according to one embodiment.
Figure 2 illustrates a flow chart of the method of the present invention according to another embodiment.

### MODES FOR CARRYING OUT THE INVENTION

### Definitions

The following terms are intended to have the following general meanings as they are used herein:

Nucleic acid - a high molecular weight material that is a polynucleotide or an oligonucleotide of DNA or RNA.

Polynucleotide - a compound or composition that is a polymeric nucleotide or nucleic acid polymer. The polynucleotide may be a natural compound or a synthetic compound. In the context of an assay, the polynucleotide can have from about 20 to 5,000,000 or more nucleotides. The larger polynucleotides are generally found in the natural state. In an isolated state the polynucleotide can have about 30 to 50,000 or more nucleotides, usually about 100 to 20,000 nucleotides, more frequently 500 to 10,000 nucleotides. It is thus obvious that isolation of a polynucleotide from the natural state often results in fragmentation. The polynucleotides include nucleic acids, and fragments thereof, from any source in purified or unpurified form including DNA, double-stranded or single-stranded (dsDNA and ssDNA), and RNA, including t-RNA, m-RNA, r-RNA, mitochondrial DNA and RNA, chloroplast DNA and RNA, DNA/RNA hybrids, or mixtures thereof, genes, chromosomes, plasmids, the genomes of biological materials such as microorganisms, e.g. bacteria, yeasts, viruses, viroids, molds, fungi, plants, animals, humans, and the like. The polynucleotide can be only a minor fraction of a complex mixture such as a biological sample. Also included are genes, such as hemoglobin gene for sickle-cell anemia, cystic fibrosis gene, oncogenes, cDNA, and the like.

Polynucleotides include analogs of naturally occurring polynucleotides in which one or more nucleotides are modified over naturally occurring nucleotides. Polynucleotides then, include compounds produced synthetically (for example, PNA as described in U. S. Patent No. 5,948,902 and the references cited therein, all of which are incorporated herein by reference), which can hybridize in a sequence specific manner analogous to that of naturally occurring complementary polynucleotides.

The polynucleotide can be obtained from various biological materials by procedures well known in the art. The polynucleotide, where appropriate, may be cleaved to obtain a fragment that contains a target nucleotide sequence, for example, by shearing or by treatment with a restriction endonuclease or other site-specific chemical cleavage method.

For purposes of this invention, the polynucleotide, or a cleaved fragment obtained from the polynucleotide, will usually be at least partially denatured or single-stranded or treated to render it denatured or single-stranded. Such treatments are well known in the art and include, for instance, heat or alkali treatment, or enzymatic digestion of one strand. For example, double stranded DNA (dsDNA) can be heated at 90-100°C for a period of about 1 to 10 minutes to produce denatured material, while RNA produced via transcription from a dsDNA template is already single-stranded.

Oligonucleotide - a polynucleotide, usually single-stranded, usually a synthetic polynucleotide but may be a naturally occurring polynucleotide. The oligonucleotide(s) are usually comprised of a sequence of at least 5 nucleotides, usually, 10 to 100 nucleotides, preferably, 20 to 60 nucleotides, more preferably, 20 to 35 nucleotides, and desirably about 25 nucleotides in length.

Various techniques can be employed for preparing an oligonucleotide. Such oligonucleotides can be obtained by biological synthesis or by chemical synthesis. For short sequences (up to about 100 nucleotides), chemical synthesis will frequently be more economical as compared to the biological synthesis. In addition to economy, chemical synthesis provides a convenient way of incorporating low molecular weight compounds and/or modified bases during specific synthesis steps. Furthermore, chemical synthesis is very flexible in the choice of length and region of target polynucleotides binding sequence. The oligonucleotide can be synthesized by standard methods such as those used in commercial automated nucleic acid synthesizers. Chemical synthesis of DNA on a suitably modified glass or resin can result in DNA covalently attached to the surface. This may offer advantages in - washing and sample handling. For longer sequences standard replication methods employed in molecular biology can be used such as the use of M13 for single-stranded DNA as described in J. Messing (1983) Methods Enzymol. 101:20-78.

Other methods of oligonucleotide synthesis include phosphotriester and phosphodiester methods (Narang, et al., (1979) Meth. Enzymol 68:90) and synthesis on a support (Beaucage, et al. (1981) Tetrahedron Letters 22:1859-1862) as well as phosphoramidate techniques (Caruthers, M. H., et al., "Methods in Enzymology," Vol. 154, pp. 287-314 (1988) and others described in "Synthesis and Applications of DNA and RNA," S. A. Narang, editor, Academic Press, New York, 1987, and the references contained therein. The chemical synthesis via a photolithographic method of spatially addressable arrays of oligonucleotides bound to glass surfaces is described by A. C. Pease, et al., Proc. Nat. Aca. Sci. USA (1994) 91:5022-5026. Unless otherwise noted, terms oligonucleotide and polynucleotide are used interchangeably.

Nucleotide - the monomeric unit of nucleic acid polymers, i.e., DNA and RNA, whether obtained from a natural source or produced synthetically, which comprises a nitrogenous heterocyclic base, which is a derivative of either a purine or pyrimidine, a pentose sugar, and a phosphate (or phosphoric acid). When the phosphate is removed, the monomeric unit that remains is a "nucleoside". Thus a nucleotide is a 5'-phosphate of the corresponding nucleoside. When the nitrogenous base is removed from the nucleotide, the monomeric unit that remains is a "phosphodiester". For the purposes of the invention, "nucleotide" includes its corresponding nucleoside and phosphodiester, and "oligonucleotide" includes its corresponding oligonucleoside and oligophosphodiester, unless indicated otherwise. The term "nucleotide" includes "modified nucleotide" that contains a modified base, sugar or phosphate group. The modified nucleotide can be produced by a chemical modification of a nucleotide either as part of the nucleic acid polymer or prior to the incorporation of the modified nucleotide into the nucleic acid polymer. For example, the methods mentioned above for the synthesis of an oligonucleotide may be employed. In another approach, a modified nucleotide can be produced by incorporating a modified nucleoside triphosphate into the polymer chain during an amplification reaction. Examples of modified nucleotides, by way of illustration and not limitation, include dideoxynucleotides, derivatives or analogs that are biotinylated, amine modified, alkylated, fluorophore-labeled, and the like and also include phosphorothioate, phosphite, ring atom modified derivatives, and so forth.

Target material or target - a sequence of nucleotides to be identified, usually existing within a portion or all of a polynucleotide, usually a polynucleotide analyte. The identity of the target nucleotide sequence generally is known to a extent sufficient to allow preparation of various probe sequences hybridizable with the target material.

The target material usually contains from about 30 to 5,000 or more nucleotides, preferably 50 to 1,000 nucleotides. The target material is generally a fraction of a larger molecule or it may be substantially the entire molecule such as a polynucleotide as described above. The minimum number of nucleotides in the target material is selected to assure that the presence of a target polynucleotide in a sample is a specific indicator of the presence of polynucleotide in a sample. The maximum number of nucleotides in the target material is normally governed by several factors: the length of the polynucleotide from which it is derived, the tendency of such polynucleotide to be broken by shearing or other processes during isolation, the efficiency of any procedures required to prepare the sample for analysis (e.g. transcription of a DNA template into RNA) and the efficiency of detection and/or amplification of the target nucleotide sequence, where appropriate.

Nucleic acid probe - an oligonucleotide or polynucleotide employed to bind to a portion of a polynucleotide such as another oligonucleotide or a target material. The design and preparation of the nucleic acid probes are generally dependent upon the sensitivity and specificity required, the sequence of the target material and, in certain cases, the biological significance of certain portions of the target material.

Hybridization (hybridizing) and binding - in the context of nucleotide sequences these terms are used interchangeably herein. The ability of two nucleotide sequences to hybridize with each other is based on the degree of complementarity of the two nucleotide sequences, which in turn is based on the fraction of matched complementary nucleotide pairs. The more nucleotides in a given sequence that are complementary to another sequence, the more stringent the conditions can be for hybridization and the more specific will be the binding of the two sequences. Increased stringency is achieved by elevating the temperature, increasing the ratio of co-solvents, lowering the salt concentration, and the like. For the purposes of the invention, hybridization of complementary Watson/Crick base pairs of probes on the microarray and of the target material is preferred, but non Watson/Crick base pairing during hybridization may also occur.

Conventional hybridization solutions and processes for hybridization are described in J. Sambrook, E. F. Fritsch, T. Maniatis, Molecular Cloning: A Laboratory Manual, Cold Spring Harbor Laboratory Press, Cold Spring Harbor, Ed. 2^{nd}, 1989, vol. 1-3, incorporated herein by reference. Conditions for hybridization typically include (1) high ionic strength solution, (2) at a controlled temperature, and (3) in the presence of carrier DNA and surfactants and chelators of divalent cations, all of which are well known in the art.

Complementary - Two sequences are complementary when the sequence of one can bind to the sequence of the other in an anti-parallel sense wherein the 3'-end of each sequence binds to the 5'-end of the other sequence and each A, T(U), G, and C of one sequence is then aligned with a T(U), A, C, and G, respectively, of the other sequence, to form Watson/Crick base pairs. RNA sequences can also include complementary G=U or U=G base pairs. Non-standard or non Watson/Crick base pairing is also possible with nucleotide complements, for instance, the sequences may be parallel to each other and complementary A=C or G=U base pairs in RNA sequences or complementary G=T or A=C base pairs in DNA sequences may occur, although are not preferred.

Substrate or surface - a porous or non-porous water insoluble support material. The surface can have any one of a number of shapes, such as strip, plate, disk, rod, particle, including bead, and the like. The substrate can be hydrophobic or hydrophilic or capable of being rendered hydrophobic or hydrophilic and includes inorganic powders such as silica, magnesium sulfate, and alumina; natural polymeric materials, particularly cellulosic materials and materials derived from cellulose, such as fiber-containing papers, e.g., filter paper, chromatographic paper, etc.; synthetic or modified naturally occurring polymers, such as nitrocellulose, cellulose acetate, poly (vinyl chloride), polyacrylamide, cross linked dextran, agarose, polyacrylate, polyethylene, polypropylene, poly (4-methylbutene), polystyrene, polymethacrylate, poly(ethylene terephthalate), nylon, poly(vinyl butyrate), etc.; either used by themselves or in conjunction with other materials; glass available as Bioglass, ceramics, metals, and the like. Natural or synthetic assemblies such as liposomes, phospholipid vesicles, and cells can also be employed.

Common substrates used for arrays in accordance with the invention are surface-derivatized glass or silica, or polymer membrane surfaces, as described in Z. Guo et al. (cited above) and U. Maskos, E. M. Southern, Nucleic Acids Res 20, 1679-84 (1992) and E. M. Southern et al., Nucleic Acids Res 22, 1368-73 (1994), both incorporated herein by reference. In modifying siliceous or metal oxide surfaces, one technique that has been used is derivatization with bifunctional silanes, i. e., silanes having a first functional group enabling covalent binding to the surface (often an Si-halogen or Si-alkoxy group, as in SiCl₃ or -Si(OCH₃)₃, respectively) and a second functional group that can impart the desired chemical and/or physical modifications to the surface to covalently or non-covalently attach ligands and/or the polymers or monomers for the biological probe array. Silylated derivatizations and other surface derivatizations that are known in the art are within the scope of the invention. See, for example, U. S. Patent No. 5,624,711 to Sundberg, U. S. Patent No. 5,266,222 to Willis and U. S. Patent No. 5,137,765 to Farnsworth, each incorporated herein by reference.

Immobilization of oligonucleotides on a substrate or surface may be accomplished by well-known techniques, commonly available in the literature. See, for example, A. C. Pease, et al., Proc. Nat. Acad. Sci. USA, 91:5022-5026 (1994); Z. Guo, R. A. Guilfoyle, A. J. Thiel, R. Wang, L. M. Smith, Nucleic Acids Res 22,-5456-65 (1994); and M. Schena, D. Shalon, R. W. Davis, P. O. Brown, Science, 270, 467-70 (1995), each incorporated herein by reference.

Siliceous substrate-Any material largely comprised of silicon dioxide. Silylated siliceous substrate is a siliceous substrate that has at least one surface derivatized with a silane compound using materials and methods known in the art to facilitate the bonding of nucleic acid probes.

### Detailed Description of the Invention

Although the inventors do not want to be held to any particular theory for the cause of the problem in the hybridization assay art, the inventors believe that the conventional hybridization conditions at the preferred high temperatures above 50°C for 6 hours or more were degrading the surface derivatization on the siliceous substrate, and further dissolving the siliceous substrate itself with time. It is known that conventional glass will dissolve in water over time. The rate of dissolution is dependent on many factors. The dissolution of glass is essentially a depolymerization through hydrolysis, which is accelerated in the presence of excess hydroxide (basic) or hydrogen (acidic) ions. Ralph K. Iler discusses silica dissolution chemistry in The Chemistry of Silica, John Wiley & Sons, N. Y., pp 62-77, incorporated herein by reference. Characteristics, such as the low-intensity or missing features on the assay substrate, high backgrounds, and/or visually "blotchy" substrates after hybridization assays appeared consistent with substrate surface dissolution.

Several conventional buffer solutions were evaluated in high temperature hybridization assays to determine their effects on siliceous substrates with time. The effects on the substrate were measured by the hybridization results achieved under high temperature conditions, and further, by the surface color (or uniformity) after the assay. It was determined that at least the buffer used and the pH of the hybridization solution played an important role in the quality of hybridization assay results and surface uniformity. Certain conventionally used buffers containing sodium phosphate (i.e., SSPE) appeared particularly deleterious to derivatized siliceous substrates at high temperature. Further, hybridization solutions maintained at a pH of about pH 6.8 and above apparently contributed to the deleterious effects. This problem impacted the performance of biological assay results in many ways. For example, the hybridized features had low-intensity or were missing from the substrate surface and array uniformity was affected if different regions of the array substrate surface showed different dissolution rates. It appeared that some of the conventional hybridization buffers at conventional solution pH were not compatible with hybridization assays at high temperature and repeatedly produced poor hybridization assay results.

The surface dissolution theory may be confirmed by comparing to the field of high performance liquid chromatograph (HPLC), where silica-based columns are used for chromatography separations. The silica-based column material is typically derivatized with various alkyl silanes, such as octadecyltrichlorosilane. A problem exists in HPLC, wherein aqueous-based solvents gradually degrade the alkyl silane layer. See for example, E. F. Vansant et al., "Characterization and Chemical Modification of the Silica Surface", p. 149-192, Elsevier, New York, 1995, incorporated herein by reference. As the alkyl silane layer is degraded, the stability of silica-based column degrades over time due to the dissolution of the silica caused by the pH of the buffer solutions and the temperatures used. J. J. Kirkland et al. discuss the stability of silica-based columns in "Stability of Silica-based, endcapped columns with pH 7 and 11 mobile phases for reversed-phase high-performance liquid chromatography", Journal of Chromatography A, 762 (1997) 97-112, incorporated herein by reference. It was reported therein that silica-based columns are not usually recommended for operation at higher pH (e.g., > pH 8), because of potential dissolution of the silica support and accompanied column failure. Moreover, problems exist in the pH range of operation of 6-8, which are also attributed to dissolution of the silica support. The solubility of silica- based supports is greatly increased in the presence of phosphate buffers, particularly at higher temperatures and higher buffer concentrations. Organic buffers, e.g., Tris, in the intermediate and higher pH range were indicated to extend the life of the silica-based columns for HPLC. Where phosphate buffers are necessary, concentrations of 10-50 mM and temperatures not exceeding 40°C at intermediate pH were suggested.

While the surface dissolution problem in the art appears analogous to a problem faced in the HPLC art, the solutions found in the HPLC art do not address the additional concern of the inventors of preserving DNA hybridization assay performance. For example, the organic buffer Tris at an intermediate and higher pH range (e.g., pH 7 - 9) exhibits poor hybridization results in hybridization assays at high hybridization temperatures above 50°C. Therefore, while the Tris buffer at intermediate and higher pH range might increase the stability of silica based columns for HPLC, it is not compatible with DNA and/or RNA hybridizations on derivatized siliceous microarray substrates at high temperature. The present invention addresses both the surface dissolution problem and assay performance. The methods of the present invention provide optimized hybridization assay performance and maintain the integrity of the derivatized surface of the siliceous substrates.

Substrates or supports used in biological assays in analytical, therapeutic and diagnostic applications using nucleic acid microarrays are as defined above. Typical substrates for microarray applications are soda lime glass, pure silica, heat resistant glasses, or other materials that are typically derivatized with a silane compound ("silylated-siliceous substrate") to improve bonding of monomers or polymers of nucleic acids to the surface, as is well known in the art. While the invention is described below for silylated-siliceous substrates, other surface derivatizations are known and are within the scope of the present invention. Advantageously, the method of the present invention does not degrade the conventional siliceous substrate materials, their surface derivatizations, or hybridization assay performance thereon at high hybridization temperatures ranging from 55°C to 70°C.

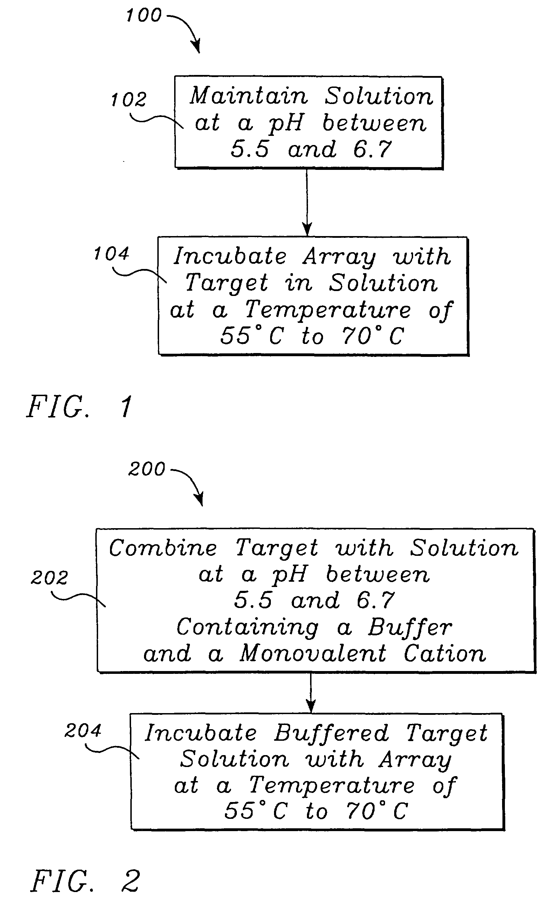

In accordance with one aspect of the invention, a method 100 of hybridizing a microarray of an oligonucleotide on a surface derivatized-siliceous substrate with another oligonucleotide materials is provided. Figure 1 is a flow chart illustrating the steps of the method 100. The method 100 comprises the steps of maintaining 102 a hybridization solution at a pH between pH 5.5 and 6.7; and incubating 104 the microarray with the oligonucleotide material in the pH-maintained hybridization solution at a temperature ranging from about 55°C to about 70°C.

In general, hybridizations conventionally can take anywhere from less than about 2 hours to more than 48 hours to be completed at hybridization temperatures ranging from about 20°C to about 70°C. However, the surface derivatized-siliceous substrate degradation problem in the art was more prevalent where higher temperatures and/or longer hybridization (incubation) times were used. Higher temperature hybridizations at about 55°C to about 70°C are known in the art to advantageously maximize the rate of Watson/Crick base-pairing than lower temperature hybridizations at about 20°C to about 50°C, and therefore, higher temperature hybridizations are preferred.

Moreover, hybridization "completion" is dependent on the user and the target oligonucleotide material to be hybridized; the hybridization time is not intended to limit the scope of the invention, but instead, the hybridization time is intended to emphasis the benefit of the invention. Hybridization could comprise anywhere from 1% to 100% of the probes on at least one feature of the microarray being hybridized with the target material, for example. The hybridization time periods to achieve completion within the 1 % to 100% range will vary greatly. The hybridization time periods at the higher temperatures usually are about 8 hours to more than about 24 hours typically, to achieve optimum results or throughput, when the nucleotide make-up of the mixture of probes is diverse and/or the target population is complex. However, the problem of surface derivatized-siliceous substrate dissolution in the art was visible in hybridizations as early as about 6 hours during high temperature hybridizations.

The present method 100 overcomes the problems of degraded siliceous surfaces during hybridizations at higher temperatures and for longer incubation time periods. In accordance with the method 100, the hybridization solution is maintained at a pH ranging from about 5.5 to 6.7 using a buffer having a buffering capacity between pH 5.5 and 6.7. By buffering capacity it is meant that the buffer has the ability to buffer or maintain a constant pH or pH range during the incubation period and at the - hybridization temperature. Several different organic buffers were evaluated based on their reported p*Ka* at 25°C and useful pH range. The following Table 1 provides two buffers having optimum buffer capacity for the present invention. The buffers listed in Table 1 are illustrative only and not intended to limit the scope of the invention. Other buffers that have a buffering capacity at a pH in the pH range of 5.5 to 6.7 and that are soluble at the concentration of interest are also within the scope of the invention. The information in Table 1 was obtained from Sigma Catalog, "Molecular Biology Reagents"; free acids that are RNase-, DNase-, and protease-free, page 1913.

**Table 1: Buffers**

| Part No. | Buffer | p*Ka* at 25C | Useful pH range |
|---|---|---|---|
| M-3023 | MES | 6.1 | 5.5 - 6.7 |
| M-8899 | MOPS | 7.2 | 6.5 - 7.9 |
| MES: 2-[N-morpholino] ethane sulfonic acid. | | | |
| MOPS: 3-[N-morpholino] propane sulfonic acid | | | |

In accordance with the invention, if the pH goes above pH 6.7, it is believed that the degradation of a silylated-siliceous substrate increases with longer incubation time at the higher temperature. As a result, less nucleic acid remains on the surface, less hybridization occurs and assay performance becomes compromised. As the pH decreases from pH 6.0 to pH 5.5, the amount of non-Watson/Crick base pairing (specifically A::C base-pairing) increases during hybridization. While non Watson/Crick base pairing may be acceptable, typically it is not preferred. Maintaining the pH preferably between pH 6.0 and 6.6 advantageously provides a high amount of Watson/Crick base pairing and minimizes silylated-siliceous substrate degradation during conventional long hybridization time periods at high hybridization temperatures. The conventional SSPE buffers, having a useful pH range typically above pH 7.0, contain sodium phosphate, which acts as a catalyst for dissolution and greatly accelerates the rate of dissolution at higher temperatures. The conventional Tris-Cl organic buffers ("Tris" is a well-known abbreviation for Tris-[hydroxymethyl] amino methane), having a p*Ka* of 8.1 at 25°C and a useful pH range of 7.0 - 9.0, also degrades the silylated-siliceous substrate surface when used in hybridization assays of nucleic acid materials having typical long incubation time periods and high hybridization temperatures. Problems start as early as about 6 hours and after about 8 hours of incubation time or more at 60°C and higher, there is little or no evidence of hybridizations when the phosphate buffers and Tris buffers are used. However, it should be noted that Tris buffers could be used below their "optimal buffering range" (i.e., in the pH range of pH 6.1-pH 6.7) and still provide reasonable assay performance for the purposes of the invention. The buffers in Table 1 are preferred since their optimal or useful buffering ranges are within the pH range of the invention. In other words, there is a higher confidence level that the buffers of Table 1 will maintain the pH in the appropriate range of pH 5.5 - 6.7 during the course of the hybridization.

More preferably, in the present method 100, the surface derivatization on the siliceous substrate comprises silane, the pH range is between 6.1 and 6.6 and the hybridization temperature ranges from about 60°C to 66°C. Most preferably, the pH range is between pH 6.1 and 6.4. Decreasing the pH from 6.6 to about 6.4 results in less hydrolytic degradation of the surface, improving the overall functional performance of the array. The present method may be used for hybridizations times ranging from less than about 2 hours to at least 48 hours. More importantly, the present invention works well where the conventional hybridization parameters fail (i.e., at the hybridization time of about 6 hours and much longer). The present invention works particularly well for hybridizations preferably taking up to at least about 24 hours, and more preferably between about 12 hours and about 24 hours.

In addition to the buffer, the hybridization solution of the present method further comprises a monovalent cation provided by a salt preferably selected from one or more of LiCl, NaCl, and KCI. Although not preferred, a quaternary alkylammonium salt may be used instead. The quaternary alkylammonium salts include, but are not limited to, one or more of tetraethylammonium chloride (TEACl) and/or tetramethylammonium chloride (TMACl). However, the effectiveness of using quaternary alkylammonium salts in the buffer of the invention to overcome the dissolution problem in the art may be limited. Quaternary alkylammonium salts are known to lower the melting temperatures (T_{*m*}*s*) of complementary nucleotide hybrids. Therefore, the optimal hybridization temperatures when using a quaternary alkylammonium salt in the buffer may be in a range where the rate of dissolution of siliceous surfaces is relatively slow. Their use and effectiveness in the invention will depend on many factors, such as concentration, oligomer length, etc. More preferably, the buffer is selected from one or more of Li-MES, Na-MES, K-MES, Li-MOPS, Na-MOPS or K-MOPS.

In another embodiment, the hybridization solution further comprises one or more of a chelating agent and/or a surfactant. Typically, the chelating agent is used to sequester divalent cations that stabilize nucleic acid tertiary structures. However, the chelating agent may also participate in degrading the silylated surface of the siliceous substrate. Therefore, whether to use a chelating agent and how much to use in the hybridization solution will depend on many factors, which one skilled in the art can determine without undue experimentation. Divalent cation chelating agents useful for the invention include, but are not limited to, EDTA (ethylenediaminetetraacetic acid), CDTA (trans-1,2-diaminocyclohexanetetraacetic acid) or DTPA (diethylenetriaminopentaacetic acid).

A surfactant is useful for providing better mixing of the hybridization solution, target material and the probes on the microarray. However, the surfactant may contribute to silylated-siliceous substrate degradation also. Therefore, whether to use a surfactant, which one to use and how much to use in the hybridization solution will depend on many factors, including but not limited to incubation time, temperature, pH and the siliceous substrate used, all of which one skilled in the art can readily determine without undue experimentation. When used in the invention, the surfactant may be selected from one or more of Triton X-102® , Triton X-100® , or a carboxylic acid salt. Triton X-102® and Triton X-100® are trademarks of Union Carbide and are octylphenoxy-polyoxyethylene ethers. The carboxylic acid salt preferably includes, but is not limited to, one or more of sodium dodecyl sulfate, N-lauryl sarcoside, acylated polypeptides, linear alkybenzene sulfonates, lignin sulfonates, paraffin sulfonates, sulfosuccinate esters, alkylnaphthalene sulfonates, isethionates, phosphoric acid esters, perfluorinated carboxylic acid salts, polyoxyethylenated alkylphenols, polyoxyethylenated straight chain alcohols, polyoxyethylenated polyoxypropylene glycols, polyoxyethylenated mercaptans, long chain carboxylic acid esters, alkanolamine condensates, and/or N-alkylpyrrolidones. It is well known, for example, that dodecyl sulfates are used to slow RNase degradation during hybridizations and therefore, have particular use when the target material or the nucleic acid probes are RNA-based. However, dodecyl sulfate surfactants may not be necessary when the target material and probes are DNA-based. Moreover, when a dodecyl sulfate surfactant is included in the hybridization solution, it is well known that using a Li+ salt will help keep the dodecyl sulfate in solution.

In another embodiment of the present invention, a method 200 of hybridizing a microarray of an oligonucleotide on a silylated-siliceous substrate with another oligonucleotide material is provided. Figure 2 is a flow chart illustrating the steps of the method 200. The method 200 of hybridizing comprises the steps combining the oligonucleotide material with a hybridization solution having a pH between pH 5.5 and 6.7 comprising a buffer and a monovalent cation; and incubating 204 the material in the buffered solution with the microarray at a hybridization temperature ranging from about 55°C to about 70°C so as to hybridize the oligonucleotide material.

In the preferred embodiment of the method 200 of hybridizing, the pH of the hybridization solution ranges from pH 6.0 to 6.7, more preferably from pH 6.1 to 6.6, and most preferably from pH 6.1 to 6.4. The buffer is selected from one or more of the buffers listed in Table 1, although other buffers with similar buffering capacity may be used as long as they are soluble at the concentration of interest. The monovalent cation is provided by a salt preferably selected from one or more of NaCl, LiCl or KCl. The incubation temperature preferably ranges from 60°C to 66°C and the incubation time to hybridize the oligonucleotide material is greater than or equal to 6 hours (e.g., to at least about 48 hours), preferably within at least 24 hours, and more preferably from about 12 hours to at least 24 hours.

As mentioned above for method 100, the hybridization solution used in method 200 preferably may further comprise one or more of a chelating agent and/or a surfactant, as described above.

Key to the present method 100, 200 is a hybridization solution having a pH within a range of between pH 5.5 and 6.7. At the lower limit between pH 5.5 and pH 6.0, more non Watson/Crick base pairing tends to occur. In accordance with the invention, non-Watson/Crick base pairing is acceptable, but not preferred. Preferably, the lower limit of the pH range is not less than pH 6.0, and more preferably, is pH 6.1, to further minimize or substantially eliminate non-Watson/Crick base pairing. At the upper limit of the pH, above pH 6.7, the amount of observable hybridizations decreases, believed to be due to an increase in silylated-siliceous surface degradation. At pH 6.6 and below, the amount of observable hybridizations increases significantly. Preferably, the upper limit in pH range is not more than pH 6.6 to further increase the amount of observable hybridizations at high temperatures, and more preferably, the upper limit is a pH of 6.4, which has shown to slow degradation of the surface, improving the overall functional performance of the array. Although a pH of less than pH 6.0 or greater than pH 6.6 provide hybridizations in accordance with the invention that are a significant improvement over conventional methods, the yield and quality of the hybridizations are not as optimum. A preferred pH for the invention is a pH within the range of pH 6.0 and 6.6, more preferably, the pH is within the range of pH 6.1 and 6.6, and most preferably the pH is within the range of pH 6.1 and 6.4, to produce optimum hybridizations at high temperatures. The present invention according to method 100, 200 advantageously can provide optimized hybridization results at conventionally high hybridization temperatures and long incubation time periods on silylated-siliceous substrates.

For the purposes of the present invention, the oligonucleotide bound to the microarray surface may be either a probe or a target under test. Further, the other oligonucleotide material may be either the probe or the target. However, when the probe is bound to the microarray, the other material is the target and where the target is bound to the microarray, the other material is the probe. Further, the target material or the probes may be directly or indirectly labeled using conventional methods, such that after hybridization, the targets or probes that are hybridized emit a signal when optically interrogated. The resulting hybridized microarray of the invention is washed to flush unhybridized material off the surface and then spun or blown dry using conventional methods. The dried microarray is optically scanned to measure the degree of hybridization using conventional scanning equipment and techniques. Where the target or probe is indirectly labeled, a post-hybridization stain, such as streptavidin covalently linked to a fluorophore or colloidal gold, is typically applied to the microarray after hybridization, but before final washing. The signal intensities and locations of the signals on the microarray provide much information about the target material.

In another aspect of the invention, a kit is provided that comprises a microarray populated with an oligonucleotide for a user. The microarray comprises a siliceous substrate with a surface derivatization to facilitate the immobilization of the oligonucleotide thereon. Preferably, the substrate is a silylated-siliceous substrate. The kit further comprises instructions to the user for performing a high temperature hybridization assay using the method 100, 200. Preferably, the kit further comprises a hybridization solution comprising a buffer having a buffering capacity at a pH within the range of pH 5.5 and 6.7, as described above for method 100, 200.

When the user receives the kit of the present invention, the user or an agent thereof (including but not limited to, a parent or a subsidiary of the parent or of the user, a contractor, subcontractor, vendor, customer, or the like) will typically expose an oligonucleotide sample to the microarray in accordance with the instructions. The hybridized array is then interrogated following exposure. Interrogation is usually accomplished by a suitable scanner that can read the location and intensity of fluorescence at each feature of an array following exposure to a fluorescently labeled sample (such as a polynucleotide containing sample). For example, such a scanner may be similar to the GENEARRAY scanner available from Hewlett-Packard, Palo Alto, CA. Results from the interrogation can be processed results, such as obtained by rejecting a reading for a feature which is below a predetermined threshold and/or forming conclusions based on the pattern read from the array (such as whether or not a particular target sequence may have been present). The hybridization assay may be performed in a first location, the interrogation may be performed in the first or a second location different from the first by a user or an agent thereof, and the results of the interrogation (processed or not) can be forwarded (such as by communication) to a third location remote from the first or second location, if desired, and received there for further use by the user or an agent thereof.

By "remote" location, it is meant that the first, second or third location is at least in a different building from the others, and may be at least one mile, ten miles, or at least one hundred miles apart. "Communicating" information references transmitting the data representing that information as electrical signals over a suitable communication channel (for example, a private or public network). "Forwarding" an item (including information) refers to any means of getting that item from one location to the next, whether by physically transporting that item or otherwise (where that is possible) and includes, at least in the case of data, physically transporting a medium carrying the data or communicating the data.

The present invention allows sensitive, selective detection of nucleic acid targets, while preserving the intactness of the surface derivatized-siliceous substrate. The method of the invention overcomes the problems found in the art of performing assays on the preferred silylated-siliceous substrates at the preferred high hybridization temperatures of between about 55°C and 70°C for long incubation times of greater than about 6 hours.

### Examples

The following examples illustrate preferred hybridization solutions having buffering capacity between pH 6.0 and 6.6 at the hybridization temperature range of 55°C and 70°C. The solutions were used in the method of hybridizing 100, 200 of the invention using arrays of oligomers attached to array siliceous substrates that were silane-derivatized. The examples herein are illustrative only and not intended to limit the scope of the invention. The components, the volumes and the final concentrations below may each vary and still be within the scope of the invention.

### Example 1 of a 2x Hybridization buffer solution in accordance with a preferred embodiment of the invention:

| Final Concentration | Component | Volume (µl) |
|---|---|---|
| | Nuclease-free water | 230 |
| 1225 mM 8.0 M LiCl | 8.0 M LiCl | 1530 |
| 300 mM | 0.5 M Li-MES (pH 6.1) | 6000 |
| 12 mM | 0.5 M EDTA (pH 8.0) | 240 |
| 3.0% (w/v) 2.0% (w/v) | 15% Lithium Dodecyl Sulfate, 10% Triton X-102 | 2000 |
| Total monovalent cation is 1500 mM | Volume 2x (ml) | 10 |

### Example 2 of a 2x Hybridization buffer solution in accordance with the preferred embodiment of the invention

| Final Concentration | Component | Volume (µl) |
|---|---|---|
| | Nuclease-free water | 398 |
| 1225 mM | 8.0 M LiCl | 1530 |
| 300 mM | 0.5 M Li-MES (pH 6.1) | 6000 |
| 36 mM | 0.5 M NaCl | 72 |
| 3.0% (w/v) 2.0% (w/v) | 15% Lithium Dodecyl Sulfate, 10% Triton X-102 | 2000 |
| Total monovalent cation is 1500 mM | Volume 2x (ml) | 10 |

Thus there has been described a new method 100, 200 and a kit for hybridizing oligonucleotides or polynucleotides on a siliceous substrate having a derivatized surface at high hybridization temperatures. It should be understood that the above-described embodiments and examples are merely illustrative of the some of the many specific embodiments that represent the principles of the present invention. Clearly, numerous other arrangements can be readily devised by those skilled in the art without departing from the scope of the present invention.

## Claims

1. A method (100, 200) of hybridizing a microarray of an oligonucleotide bound to a surface derivatized siliceous substrate with another oligonucleotide material comprising the steps of:
maintaining (102) a hybridization solution at a pH between
pH 5.5 and 6.7 with a
buffer having buffering capacity between pH 5.5 and 6.7 and a monovalent cation; and
incubating (104, 204) the microarray with the oligonucleotide material in the pH maintained hybridization solution at a hybridization temperature ranging from about 55°C to about 70°C.

2. The method (100, 200) of Claim 1, wherein the buffer is selected from one or more of 2-[N-morpholino] ethane sulfonic acid (MES) or 3-[N-morpholino] propane sulfonic acid (MOPS) and the monovalent cation is provided by a salt selected from one or more of LiCl, NaCl, KCI, or a quaternary allcylammonium salt.5

3. The method (100, 200) of any of Claims 1-2, wherein the pH is maintained (102) between pH 6.0 and 6.6.

4. The method (100, 200) of any of Claims 1-3, wherein the pH is maintained (102) between pH 6.1 and 6.6.

5. The method (100, 200) of any of Claim 1-4, wherein the pH is maintained (102) between pH 6.1 and 6.4 and the microarray and the oligonucleotide material are incubated (104, 204) for a time period ranging from less than about 2 hours to at least 48 hours in the pH-maintained hybridization solution.

6. The method (100, 200) of any of Claims 1-5, wherein the hybridization temperature ranges from about 60°C to 66°C.

7. The method (100, 200) of any of Claims 1-6, wherein the hybridization solution further comprises one or both of a chelating agent and a surfactant.

8. The method (100, 200) of any of Claims 1-7, wherein the surface of the siliceous substrate is silane-derivatized.

9. The method according to any of Claims 1-8, after the step of incubating, further comprising one or more of the steps of:
interrogating the hybridized microarray at a first location;
transmitting data representing a result of the interrogation; and
receiving the transmitted data at a second location, wherein the first location is the same or remote from the second location.

10. The method (100, 200) of any of Claims 1-9 used in a kit for hybridizing an oligonucleotide material comprising:
a microarray having a sifceous substrate, wherein a surface of the substrate is derivatized, and a plurality of an oligonucleotide attached to the surface in an array pattern of features; and
instructions for using the kit with the oligonucleotide material comprising the method.
